Europäisches Patentamt

European Patent Office

Office européen des brevets

(19)

(11) Veröffentlichungsnummer: **0 236 855**
**A1**

(12) # EUROPÄISCHE PATENTANMELDUNG

(21) Anmeldenummer: 87102677.9

(22) Anmeldetag: 25.02.87

(51) Int. Cl.4: **A61K 31/19** , A61K 31/195 , A61K 9/14 , A61K 9/08 , //A61K31/51,A61K31/44,A61K3-1/68

(30) Priorität: 06.03.86 DE 3607339

(43) Veröffentlichungstag der Anmeldung:
16.09.87 Patentblatt 87/38

(84) Benannte Vertragsstaaten:
AT CH DE LI

(71) Anmelder: Merck Patent Gesellschaft mit beschränkter Haftung
Frankfurter Strasse 250
D-6100 Darmstadt(DE)

(72) Erfinder: Wotschokowsky, Manfred, Dr.
Langgässerweg 52
D-6100 Darmstadt(DE)
Erfinder: Loebich, Friedrich, Dr.
Heinrich-Delp-Strasse 136
D-6100 Darmstadt(DE)
Erfinder: Starz, Karola
Beckstrasse 4
D-6100 Darmstadt(DE)
Erfinder: Krüger, Ludwig
Ruhlandstrasse 75
D-8750 Aschaffenburg(DE)

(54) Pharmazeutische Zubereitung.

(57) Die Erfindung betrifft eine neue pharmazeutische Zubereitung in Form eines Lyophilisats enthaltend Diclofenac-Na, dadurch gekennzeichnet, daß sie eine physiologisch unbedenkliche Aminosäure, eine matrixbildende Substanz und eine solche Menge einer physiologisch unbedenklichen Base enthält, daß sie mit Wasser eine Lösung mit einem pH-Wert von 8 bis 10 bildet.

EP 0 236 855 A1

## Pharmazeutische Zubereitung

Die Erfindung betrifft eine pharmazeutische Zubereitung in Form eines Lyophilisats, das Diclofenac-Na [I, Natriumsalz der 2-(2,6-Dichloranilino)-phenylessigsäure] enthält.

I ist in Wasser nur mäßig löslich; die gesättigte Lösung besitzt einen pH-Wert von ca. 7. Mit abnehmendem pH-Wert solcher Lösungen nimmt die Löslichkeit von I ab. Die entsprechende Säure ist praktisch in Wasser unlöslich. Ampullenpräparate von I mit schwach alkalischem pH-Wert (ca. 8,5) sind bereits bekannt.

Der Erfindung lag die Aufgabe zugrunde, injizierbare I enthaltende pharmazeutische Zubereitungen aufzufinden, mit deren Hilfe es möglich ist, eine ausreichende Menge I auch in einer Lösung mit niedrigerem pH-Wert zu verabfolgen. Insbesondere war es Aufgabe der Erfindung, eine pharmazeutische Zubereitung zur Verfügung zu stellen, mit deren Hilfe es gelingt, eine ausreichende Menge I zusammen mit Vitaminen der B-Gruppe ($B_1$, $B_6$ und/oder $B_{12}$) zu applizieren.

Mit den bekannten Ampullenpräparaten kann diese Aufgabe nicht gelöst werden, denn bei pH-Werten um 8,5 sind die Vitamine $B_1$ und $B_{12}$ nicht stabil, und bei niedrigeren pH-Werten ist für I bzw. Diclofenac keine ausreichende Löslichkeit mehr gegeben.

Es wurde gefunden, daß diese Aufgabe gelöst werden kann, indem man zunächst eine pharmazeutische Zubereitung von I in Form eines Lyophilisats herstellt, die eine physiologisch unbedenkliche Aminosäure, eine matrixbildende Substanz und eine solche Menge einer physiologisch unbedenklichen Base enthält, daß sie mit Wasser eine Lösung mit einem pH-Wert von 8 bis 10 bildet.

Gegenstand der Erfindung sind die genannte pharmazeutische Zubereitung sowie ein Verfahren zu ihrer Herstellung, dadurch gekennzeichnet, daß man eine physilogisch unbedenkliche Aminosäure, eine matrixbildende Substanz und Diclofenac-Na in einer wäßrigen Lösung einer physiologisch unbedenklichen Base löst, wobei die Menge der Base so bemessen ist, daß die entstehende Lösung einen pH-Wert zwischen 8 und 10 besitzt, und daß man anschließend die Lösung sterilfiltriert und lyophilisiert.

Gegenstand der Erfindung ist weiterhin die Verwendung dieser pharmazeutischen Zubereitung zur Herstellung einer Injektionslösung, insbesondere dadurch gekennzeichnet, daß man die Zubereitung mit einer Lösung von Salzen der B-Vitamin-Gruppe mischt, so daß die entstehende Lösung einen pH-Wert zwischen 7 und 8,5 besitzt.

Das Lyophilisat enthält vorzugsweise 50-100, insbesondere 70-80 besonders bevorzugt 75 mg I, ferner vorzugsweise 50-500, insbesondere 100-300, besonders bevorzugt 200 mg einer oder mehrerer physiologisch unbedenklicher Aminosäure(n), sowie vorzugsweise 20-500, insbesondere 50-200, besonders bevorzugt 100 mg einer oder mehrerer matrixbildender Substanz(en).

Als Aminosäuren eignen sich insbesondere die natürlichen Aminosäuren wie Glycin, Alanin, Valin, Leucin, Isoleucin, Serin, Threonin, Asparagin, Methionin, ferner z.B. 2-Aminobuttersäure. Glycin ist besonders bevorzugt.

Als matrixbildende Substanzen (Gerüstbildner) eignen sich alle physiologisch unbedenklichen Stoffe, die dem Lyophilisat-Kuchen die für eine - schnelle Lösung erforderliche lockere Struktur geben, z.B. Polysaccharide wie Dextran, Zucker wie Saccharose oder Lactose, Zuckeralkohole wie Sorbit oder Mannit, Cellulosederivate wie Hydroxyethyl-, Hydroxypropyl-oder Hydroxypropylmethylcellulose, ferner Polyvinylpyrrolidon, Gluconsäuresalze wie Calciumgluconat, Gelatine. Dextran ist besonders bevorzugt.

Als Base ist Natriumhydroxide bevorzugt. Geeignet sind ferner Natriumcarbonat oder Natriumbicarbonat.

Die Herstellung des Lyophilisats erfolgt in üblicher Weise. Zweckmäßig löst man die Base, die Aminosäure und die matrixbildende Substanz nacheinander unter Rühren in Wasser bei Temperaturen zwischen 10 und 80, vorzugsweise 30 und 50°, gibt dann I hinzu, rührt bis zur Lösung, sterilfiltriert und lyophilisiert.

Das erhaltene Lyophilisat ist wegen des großen erforderlichen Volumens von ca. 10 ml wäßriger Lösung pro Dosis und wegen des hohen pH-Wertes als solches für einen Einsatz als Monopräparat nicht geeignet. Man kann es aber in einer sauren Lösung auflösen, so daß die entstehende Lösung einen pH-Wert zwischen 7 und 8,5 besitzt, der für eine Injektionslösung geeignet ist. Dabei kann man einen oder mehrere organische Lösungsvermittler zusetzen, z.B. 1,2-Propandiol, ferner ein oder mehrere Konservierungsmittel, z.B. Benzylalkohol.

Besonders vorteilhaft ist es jedoch, wenn man die Zubereitung mit einer wäßrigen Lösung von Salzen der B-Vitamin-Gruppe mischt, die selbst einen schwach sauren pH-Wert (etwa 3 bis 5) besitzt. So eignet sich z.B. dafür eine Lösung, die - (pro Dosis) 90-130 mg eines Vitamin-$B_1$-Salzes, z.B. Thiaminiumdichlorid oder Thiaminiumnitrat, 80-120 mg eines Vitamin-$B_6$-Salzes, z.B. Pyridoxolhydrochlorid und 0,5-2 mg eines Vitamin-$B_{12}$-Derivats, z.B. Cyanocobalamin oder Hydroxocobalamin, enthält und die weitere Zusatzstoffe enthalten kann, z.B. Konservierungsmittel wie Benzylalkohol, Basen wie Diethanolamin zur Pufferung, Lokalanästhetika wie Lidocain oder dessen Salze.

Eine bevorzugte Ausführungsform der Erfindung besteht darin, daß man das Lyophilisat in einer handelsüblichen 3-ml-Ampulle auflöst, die eine derartige Vitamin-B-Salz-Lösung enthält, z.B. Neurobion® (pH-Wert 3,7-4,0). Die Vorteile dieser AUsführungsform bestehen darin, daß sowohl das Lyophilisat als auch die Ampulle gute Langzeitstabilitäten besitzen, die resultierende Injektionslösung gut verträglich ist und ein niedriges Injektionsvolumen besitzt und daher insbesondere für eine intramuskuläre Applikation geeignet ist.

Beispiel

(a) In 8 1 Wasser für Injektionszwecke von 40 °C werden nacheinander 110 g 32 %ige Natronlauge, 200 g Glycin und 100 g Dextran 40 gelöst. Man trägt 75 g I ein, rührt bis zur Lösung, füllt mit Wasser für Injektionszwecke auf 10 1 auf und mischt. Die erhaltene Lösung (pH 9,0-9,3) wird sterilfiltriert. Je 10 ml werden in 20-ml-Injektionsgläser abgefüllt und lyophilisiert. Man erhält einen weißen kompakten Lyophilisatkuchen.

(b) Das nach (a) erhaltene Produkt wird in 3 ml einer wäßrigen Lösung, die 110 mg Thiaminiumdichlorid, 100 mg Pyridoxolhydrochlorid, 1,25 mg Vitamin $B_{12}$, 8,65 mg Diethanolamin und 30 mg Benzylalkohol enthält, gelöst. Die Lösung enthält 75 mg I und zeigt einen pH-Wert von 7,5-8,0. Sie wird unmittelbar für Injektionszwecke verwendet.

**Ansprüche**

1. Pharmazeutische Zubereitung in Form eines Lyophilisats enthaltend Diclofenac-Na, dadurch gekennzeichnet, daß sie eine physiologisch unbedenkliche Aminosäure, eine matrixbildende Substanz und eine solche Menge einer physiologisch unbedenklichen Base enthält, daß sie mit Wasser eine Lösung mit einem pH-Wert von 8 bis 10 bildet.

2. Verfahren zur Herstellung einer pharmazeutischen Zubereitung in Form eines Lyophilisats enthaltend Diclofenac-Na, dadurch gekennzeichnet, daß man eine physiologisch unbedenkliche Aminosäure, eine matrixbildende Substanz und Diclofenac-Na in einer wäßrigen Lösung einer physiologisch unbedenklichen Base löst, wobei die Menge der Base so bemessen ist, daß die entstehende Lösung einen pH-Wert zwischen 8 und 10 besitzt, und daß man anschließend die Lösung sterilfiltriert und lyophilisiert.

3. Verwendung einer pharmazeutischen Zubereitung nach Patentanspruch 1 zur Herstellung einer Injektionslösung.

4. Verwendung einer pharmazeutischen Zubereitung nach Patentanspruch 1 zur Herstellung einer Injektionslösung, dadurch gekennzeichnet, daß man die Zubereitung mit einer Lösung von Salzen der B-Vitamin-Gruppe mischt, so daß die entstehende Lösung einen pH-Wert zwischen 7 und 8,5 besitzt.

| EINSCHLÄGIGE DOKUMENTE | | | EP 87102677.9 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| X | EP - A1 - 0 109 036 (MERCK & CO. INC)<br><br>* Zusammenfassung; Ansprüche 1,6; Seite 7, Zeilen 1-3; Seite 8, Zeilen 1-6,26-33; Seite 24, Zeilen 1-8; Seite 25, Zeilen 4-11,33 - Seite 26, Zeile 5 *<br><br>-- | 1,3 | A 61 K 31/19<br>A 61 K 31/195<br>A 61 K 9/14<br>A 61 K 9/08<br>//A 61 K 31/51<br>A 61 K 31/44<br>A 61 K 31/68 |
| Y | DE - A1 - 2 925 009 (BASF)<br><br>* Ansprüche 1,2,4 *<br><br>-- | 1-3 | |
| Y | EP - A2 - 0 136 470 (MERCKLE GMBH)<br><br>* Ansprüche 1,5; Seite 4, Zeilen 9-25; Beispiele 1-3 *<br><br>-- | 1-3 | |
| A | DE - B2 - 2 438 703 (LAUVEN E.)<br><br>* Anspruch; Spalte 3, Zeilen 2-14, 23-29,47-34; Spalte 4, Zeilen 52-58; Spalte 6, Zeilen 3-8 *<br><br>-- | 1-4 | RECHERCHIERTE SACHGEBIETE (Int. Cl.4) |
| A | E.BINDER et al. "Austria-Codex Fachinformation 1984/85"<br><br>ÖSTERREICHISCHER APOTHEKER-VERLAG Wien, 1984<br><br>* Seite 1179, "Voltaren-Ampullen" *<br><br>-- | 1-4 | A 61 K 31/00<br>A 61 K 9/00 |

Der vorliegende Recherchenbericht wurde für alle Patentansprüche erstellt.

| Recherchenort | Abschlußdatum der Recherche | Prüfer |
|---|---|---|
| WIEN | 09-06-1987 | MAZZUCCO |

**Europäisches Patentamt**

# EUROPÄISCHER RECHERCHENBERICHT

## EINSCHLÄGIGE DOKUMENTE

EP 87102677.9

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | <u>AT – B – 345 447</u> (BYK GULDEN LOMBERG CHEMISCHEN FABRIK GMBH)<br><br>* Ansprüche 1,2; Seite 3, Zeilen 4,5 *<br><br>-- | 1-4 | |
| A | <u>GB – A – 1 180 574</u> (M.ISRAEL)<br><br>* Anspruch 1 *<br><br>-- | 1-4 | |
| A | <u>US – A – 2 748 054</u> (JURIST A.E.)<br><br>* Ansprüche 1,3-9; Beispiele 1-5 *<br><br>-- | 1-4 | |
| A | <u>EP – A2 – 0 152 379</u> (CIBA-GEIGY AG)<br><br>* Ansprüche 1,8,9; Beispiele 11,12; Seite 18, Zeilen 1-3; Seite 29, vorletzter Absatz *<br><br>---- | | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| WIEN | 09-06-1987 | MAZZUCCO |